# EUROPEAN PATENT SPECIFICATION

(11) **EP 4 452 373 B1**
(45) Date of publication and mention of the grant of the patent: **11.02.2026**
(21) Application number: 22840134.5
(22) Date of filing: 19.12.2022
(51) Int. Cl.: A61M 15/00

(54) **POWDER INHALER**
PULVERINHALATOR
INHALATEUR DE POUDRE

(30) Priority: 21.12.2021 EP 21216553
(43) Date of publication of application: 30.10.2024
(73) Proprietor: Chiesi Farmaceutici S.p.A., 43122 Parma (IT)
(72) Inventor: DI CASTRI, Marco, 43122 Parma (IT); MULAS, Giuseppe Antonio, 43122 Parma (IT); TWEEDIE, Alan, 43122 Parma (IT); COLLINGS, Ralph Donald Quentin, 43122 Parma (IT); HIGGINS, Daniel David, 43122 Parma (IT); RICHARDS, Benjamin Michael, 43122 Parma (IT); VARVOUNIS, Odysseas Michail, 43122 Parma (IT)
(74) Representative: PGA S.p.A.
(86) International application number: PCT/EP2022/086687
(87) International publication number: WO 2023/117929

(56) References cited:
- WO-A1-97/00703
- WO-A2-2008/074441
- US-A- 5 524 613
- US-A- 6 065 471
- US-A1- 2002 148 469
- US-A1- 2006 283 445
- US-A1- 2014 000 602
- US-B1- 6 321 747
- US-B1- 7 207 330
- US-B2- 11 071 835

## Description

### Technical field of the invention

The present invention relates to a dry powder inhaler, i.e. a device for dispensing a powdered medicament preparation by inhalation. The device is in particular a portable, multiple-dose, breath activated dry powder inhaler without propellant gas, equipped with a metering device which dispenses doses from a medicament container.

### Background art

Inhalers are hand-held portable devices that deliver medication directly to the lungs. One class of inhalers is passive dry powder inhalers ("DPI"). A passive DPI is a patient driven device wherein the action of breathing in through the device draws the powder formulation of a medicament into the respiratory tract. DPIs are well recognized as devices for drug delivery to the lungs for treatment of pulmonary and systemic diseases. They can generally be divided in: i) single-dose (unit-dose) inhalers, for the administration of an individual dose of the active ingredient/s contained in capsule or blister loaded into the device and punctured by the patient immediately before use; ii) pre-metered multi-dose inhalers containing a series of blisters or capsules with the active ingredient/s formulation or iii) reservoir inhalers containing a larger amount of the powder formulation of active ingredient/s, corresponding to multiple doses, which is metered from a storage unit just before inhalation.

Document WO 2004/012801, by the same Applicant, discloses a powder inhaler comprising a casing with a lower shell and an integral cover pivotably coupled to the lower shell. The lower shell delimits a mouthpiece and the integral cover is movable between a closed position in which the mouthpiece is enclosed and hidden by the integral cover and an open position in which the mouthpiece is exposed for use. The lower shell houses a container for storing a powdered medicament, a metering member having a dosing recess to be filled with a dose of the powdered medicament and an inhalation channel in communication with the mouthpiece. The metering member is moveable between a filling position, in which the dosing recess is in alignment with an opening of the container so as to be filled with a dose of the powdered medicament, and an inhalation position, in which the dosing recess is in alignment with an inhalation channel for enabling inhalation of the dose of the powdered medicament contained in the dosing recess. The powder inhaler further comprises a protective member which is slidingly moveable on the metering member between a closed position, in which the protective member covers the dosing recess of the metering member if the metering member is in an inhalation position, and an open position, in which the protective member exposes the dosing recess thereby enabling inhalation of the dose of the powdered medicament contained in the dosing recess. The protective member is coupled to an inhalation actuated mechanism in such a manner that the inhalation actuated mechanism moves the protective member from its closed position to its open position if there is an inhalation suction force exerted by a user which exceeds a predetermined level. Document WO 2016/000983, by the same Applicant, discloses a powder inhaler similar to the one of WO 2004/012801.

A formulation for powder inhalers is commonly a powder blend of one or more active ingredients dispersed in a pharmacologically inert bulk solid, comprising a physiologically acceptable diluent, such as lactose, and optional additional excipients such as lubricants. The particle size of inhalable active ingredients should be optimized to deliver the drug deep into the lung to achieve efficacy. This efficacious particle size typically lies between 1-6 µm whereas larger particles, in the range 6-10 µm, tend to be deposited in the upper airways without reaching the site of action in the lower airways.

It is well known that stability of the powder as well as the aerosol performances could be affected by environmental conditions, humidity in particular. Therefore, it is desirable to control the humidity within powder inhalers and in particular in reservoir DPIs. There is the need of more efficacious systems to protect from humidity, in particular but not solely, when the drug-containing powder formulation from reservoir DPIs are intended for being stored and used in sub-tropical and tropical countries. Powder inhalers provided with sealing devices are known.

For instance, document EP2063940B1 discloses an inhalation device for powder drugs comprising a storage chamber for accommodating a plurality of drug powder doses and a dosing device which includes at least one dosing slider which is movable with a translatory movement in a dosing slider passage from a filling position into an emptying position. The dosing slider passage has open end with an opening through which the dosing slider can issue with the dosing cavity, wherein a contact surface for a seal is provided around the opening and wherein the dosing slider further has a sealing surface which is arranged in a plane in approximately transverse relationship with the direction of movement out of the filling position into the emptying position. The seal may comprise a thermoplastic elastomer or a sealing rib which may be formed on the dosing slider passage or in the dosing slider. Document EP1231964B1 discloses a powder inhaler comprising a powder container, an air channel, a metering member equipped with a dosing recess, an actuating means for the displacement of the metering member between the filling and the inhalation position, and a closure element for plugging the air channel in a substantially water-proof manner when the metering member is in the filling position and opening the air channel when the metering member is in the inhalation position. Document WO2008074441A2 discloses an inhaler for dispensing a dose of an active agent to the respiratory tract of a patient, which inhaler comprises a housing and a mouthpiece attached to the housing. The housing includes storage means for storing the active agent and an airway for delivering the dose from the storage means to the mouthpiece in a stream of air for administration to the patient. A movable dosing slide is located inside the housing and is movable between a receiving position and a dispensing position. The dosing slide includes a dosing cavity positioned underneath a funnel outlet of a medicament reservoir when the dosing slide is in the receiving position. The dosing cavity is surrounded by a radial sealing rim on the underside.

Document US6065471A1 discloses an inhalation device for inhalation of a powder medicament. The device comprises a body defining a reservoir for medicaments in powder form, an outlet through which a user can inhale and a dosing member with a metering recess formed therein. The dosing member is moveable between a position in which the metering recess communicates with the reservoir to receive a dose of powder therefrom and a position in which the metering recess communicates with the outlet to permit the user to inhale the dose. A pharmaceutical grade rubber sealing ring is incorporated between a cover and the body to prevent ingression of moisture into the reservoir.

Document US5524613A1 discloses a powdered pharmaceutical inhaler including a source of pressurized gas, a pharmaceutical transfer assembly and a mouthpiece assembly. The pharmaceutical transfer assembly includes a pharmaceutical reservoir containing undivided supplies of a pharmaceutical. Quantities of the pharmaceutical are transferred from the pharmaceutical reservoir to an entrainment region along a flow path through the inhaler by one or more dosing members. The pharmaceutical transfer assembly includes a dosing plate assembly, and a resilient gasket provides a seal with surfaces of dosing plates.

Document US11071835B2 discloses a dry powder inhaler which includes: a reservoir containing a dry powder formulation and a dose metering system for delivering a metered dose of the formulation from the reservoir; a cyclone deagglomerator for breaking up agglomerates of the dry powder formulation; a delivery passageway for directing an inhalation-induced air flow through a mouthpiece. The dose metering system includes a cup assembly including a medicament cup adapted to receive formulation and mounted in a sled slidably received in a slide channel. A sealing spring is provided between the sled and the cup for biasing the medicament cup against a bottom surface of a hopper to seal a dispenser port of the reservoir.

Document US6321747B1 discloses an inhalation device comprising a body defining a reservoir for medicament in the form of a powder, an outlet through which a user can inhale, and a dosing member with a metering recess formed therein. The dosing member is moveable between a first position in which the metering recess communicates with the reservoir to receive a dose of powder therefrom and a second position in which the metering recess communicates with the outlet to permit the user to inhale the dose. The metering recess is formed in a face of the dosing member which is urged into contact against a similar mating face of the body at the lower end of the reservoir to form a dynamic seal.

Document US7207330B1 discloses a medicament delivery device which comprises a medicament reservoir, a medicament delivery passage and a metering member adapted to transfer a measured dose of medicament from the medicament reservoir to the delivery passage the device is provided with a moisture proof barrier. Sealing means comprise a resilient sealing member positioned at the end of the reservoir adjacent the metering member.

Document US20020148469A1 discloses a dispensing system including a vessel having an exit port in a first end wall and an open second end, a dispensing member capable of adopting a receiving position at which the exit port is open and discharge position at which the exit port is closed. A slide plate has an upper surface which is substantially flat and in sealing contact with the first end wall of the vessel. Sealing contact is maintained by virtue of sealing spring which urges the upper surface of slide plate against the first end wall of the hopper.

Document US2006283445A1 discloses a powder medicine administering device including a main body, an air supply section, and a movable member slidably attached to the main body and arranged to be moved relative to the main body from a first position through a second position to a third position. Main body and a slider are made from resin material having elasticity to ensure air tightness between abutment surfaces and to restrict leakage of the powder medicine.

### Summary

It is an object of the present invention to provide a powder inhaler with a further improved protection from environmental conditions, like humidity, temperature, light, pressure.

In particular, it is an object of the present invention to provide a powder inhaler which is able to control the humidity inside the inhaler itself.

It is object of the present invention to prevent or at least to limit the entry of atmospheric moisture inside the inhaler and in particular inside the container storing the powdered medicament during distribution and storage at a pharmacy (i.e. before delivering the inhaler to the user) and also after delivering the inhaler to the user, during the time period (e.g. days, months) when the user makes use of the inhaler. It is also object of the present invention to prevent dispersion the powdered medicament contained in the container.

The present invention is defined by the appended claims.

The invention allows to improve the stability of the powder and the aerosol performances (i.e. the capability of delivering the drug deep into the lung to achieve efficacy) by protecting said powder from the environmental conditions, particularly from humidity.

In particular, the invention allows to prevent or at least reduce moisture entry through gaps between the shuttle and the container when the shuttle is in the filling position.

Therefore, the invention allows to protect the powder during the period when the inhaler is in use but is closed and stored in a bag, in a pocket, in the open air, in a drawer or in a furniture or other place between inhalations.

The invention allows also to prevent or at least reduce powdered medicament losses through gaps between the shuttle and the container.

The word "less stiff" referred to the deformable portion in this description and claims means that, when the spring pushes the dosing part, the deformable portion deforms more than the dosing part and the main part, so as to allow the dosing part to slightly move towards the opening and to join to the container at the coupling zone while the main part remains in its place. In other words, the deformable portion is more flexible than the dosing part and the main part, so that the connection between the dosing part and the main part does not prevent the dosing part from adapting its position to the shape and position of the container and allows a zone surrounding the dosing recess to adhere to an edge of the opening.

### Description of the drawings

Fig.1 shows a 3D view of a powder inhaler according to the present invention in an open configuration;
Figures 2A, 2B and 2C are section views of the powder inhaler of figure 1 in different states;
Figures 3 and 4 show 3D views of one element of the powder inhaler of figure 1;
Fig.5 is a plan view of one side of the element of figure 3;
Fig.6 is a side section view of the element of figure 3;
Fig.7 is 3D view of another element of the powder inhaler of figure 1;
Fig.8 is a plan view of the element of figure 7;
Fig.9 is a section view of a powder inhaler according to the present invention provided with a variant of the element of figures 3 to 6;
Figures 10, 11 and 12 show 3D views of the variant element of figure 9;
Fig.13 is a side section view of the element of figures 10, 11, 12;
Fig.14 is a section view of a different embodiment of a powder inhaler not part of the present invention;
Fig.15 is an exploded view of some components of the inhaler of Figure 14;
Fig. 16 shows a 3D view of one element of the powder inhaler of figures 14 and 15;
Fig.17 is an enlarged section view of a portion of the element of Figure 16;
Fig.18 is a chart showing a differential weight per day, with respect to a standard powder inhaler, due to moisture absorption of the different embodiments/variants;
Fig.19 is a chart showing a cumulative weight over time due to moisture absorption in a standard powder inhaler compared with the embodiments/variants.

### Detailed description

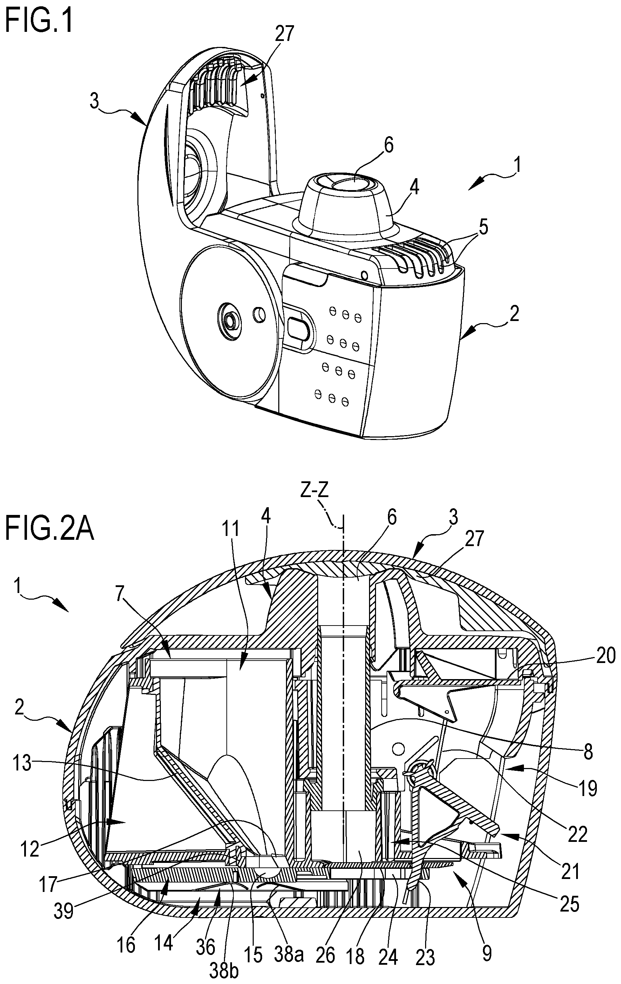

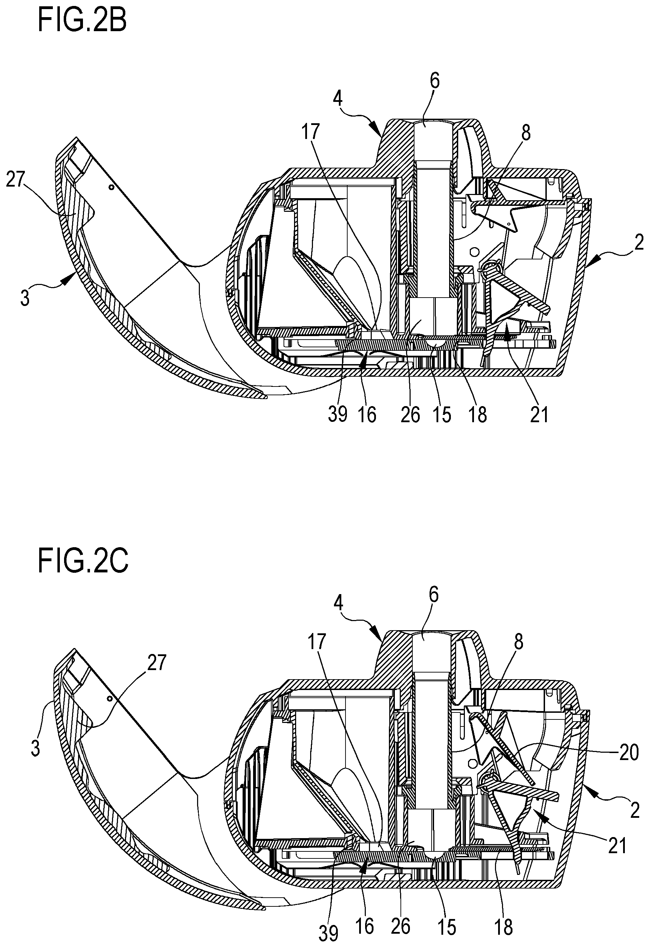

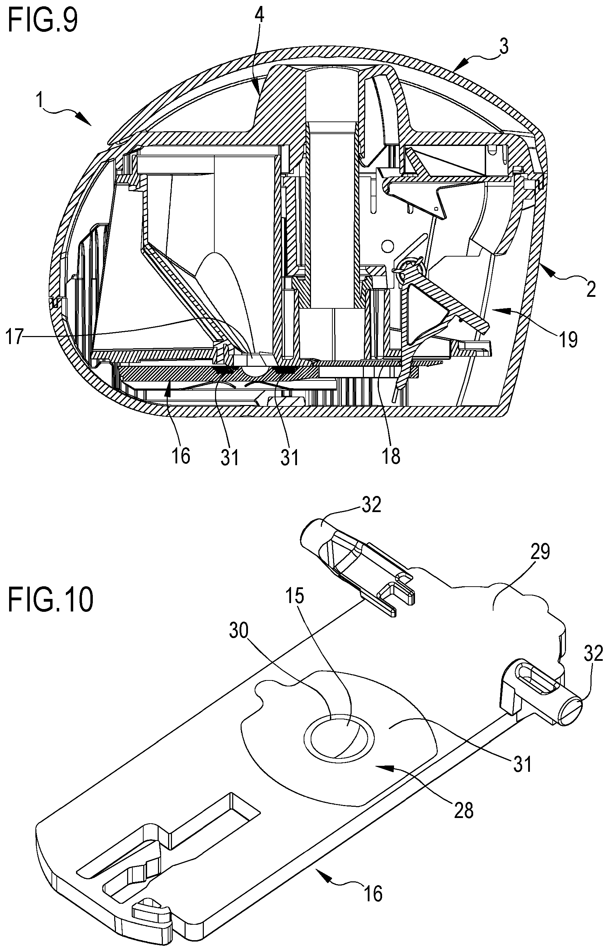

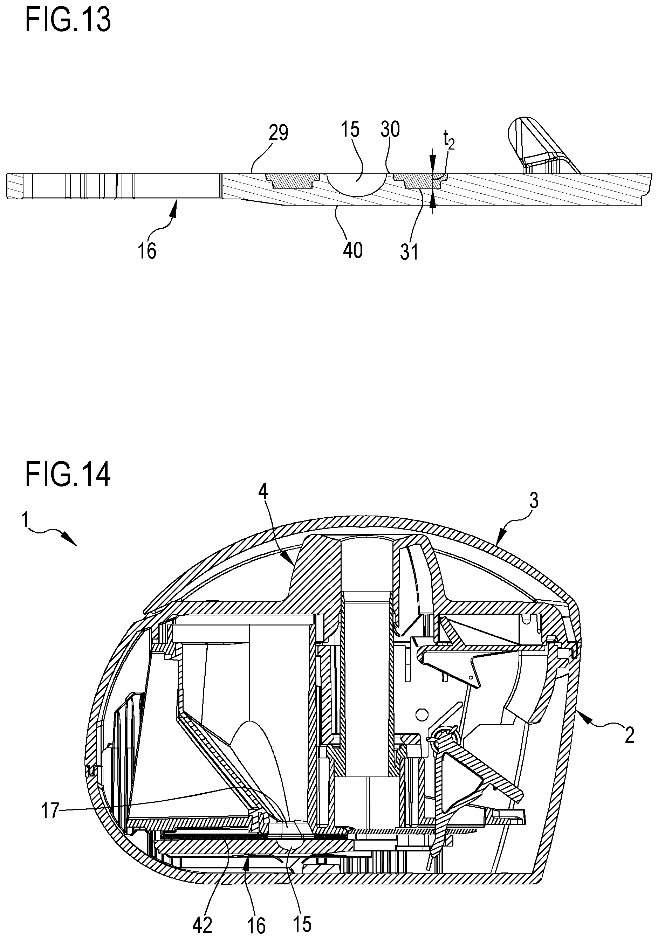

With reference to the appended drawings, Figures 1, 2A, 2B, 2C show a first embodiment, Figure 9 shows a variant of the first embodiment and Figure 14 shows an embodiment of a powder inhaler 1 not part of the present invention. The powder inhaler 1 of these non-limiting examples may be similar to the inhalers disclosed in document WO 2004/012801, WO 2016/000983, WO2021/105440 by the same Applicant.

The powder inhaler 1 comprises a casing 2 and a cover 3 being pivotably or rotatably coupled to the casing 2. As can be taken from Fig. 1, the cover 3 can be opened to reveal a mouthpiece 4 through which a user can inhale a powdered medicament. At an upper front side of the mouthpiece 4, air inlets 5 are formed in the casing 2. The casing 2 is a closed shell made of thermoplastic material (e.g. ABS) and comprises lateral sides, an upper side and a lower side (upper and lower with respect to the orientation of the powder inhaler 1 of figures 1, 2A - 2C, 9 and 14). The mouthpiece 4 protrudes from the upper side and has an external shape of truncated cone tapering towards an opening 6 fashioned in a top portion (smaller base) of the mouthpiece 4.

The cover 3 is hinged to the casing 2 and can be rotated between a closed position, shown in Figures 2A, 9 and 14 in which the cover 3 encloses the mouthpiece 4, and an open position, shown in Figures 1, 2B and 2C, in which the cover 3 is spaced from the mouthpiece 4 to expose said mouthpiece 4 for use.

The powder inhaler 1 comprises a container 7 for storing a powdered medicament, an inhalation channel 8 connected to the opening 6 of the mouthpiece 4 and a dispensing device 9. The inhalation channel 8 has a first opening connected to the mouthpiece 4 and a second opening, opposite with respect to the first opening.

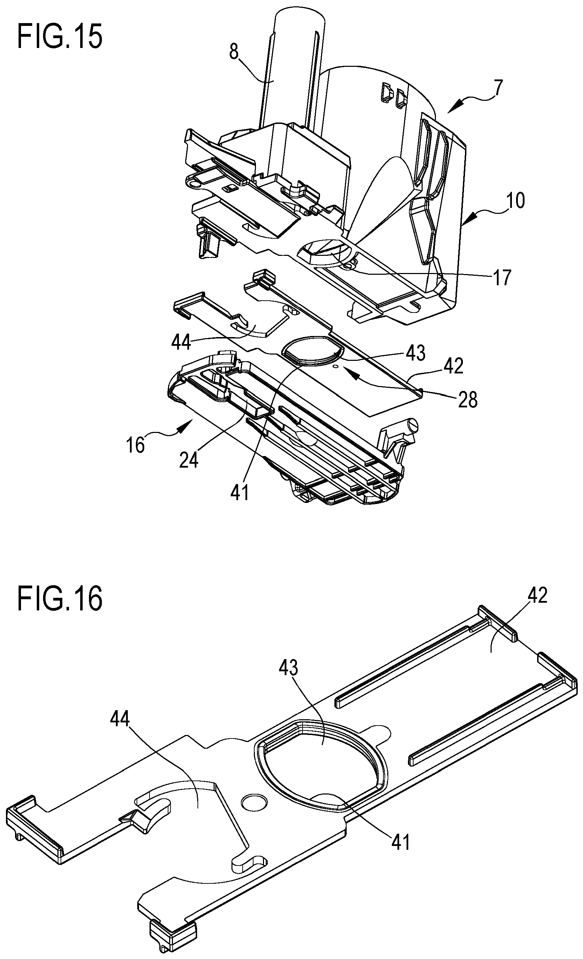

All these elements are part of sub-assembly 10, shown in Figure 15, housed inside the casing 2.

As is shown in Figures 2A-2C, 9 and 14, the container 7 is a container with integral desiccant. The container 7 comprises a medicament chamber 11 storing the powdered medicament and a desiccant chamber 12 storing a desiccant for absorbing moisture that may have entered the medicament chamber 11.

The desiccant chamber 12 is separated from the medicament chamber 11 by a separate permeable membrane 13. This permeable membrane 13 is of a different permeability than the permeability between either the desiccant or the medicament to the outside environment. The permeability of the membrane 13 can be achieved, for example, by making it of a different material and/or a thinner section than the main body of the container 7. Foils may be used to seal both the medicament chamber 11 and the desiccant chamber 12.

The container 7, in particular the medicament chamber 11, is filled or is configured to be filled with an amount of powdered medicament corresponding to a plurality of doses, e.g. up to 100 - 200 doses. For instance, the powdered medicament is a pharmaceutical composition comprising a pharmaceutically acceptable salt of formoterol, e.g. in combination with a pharmaceutically acceptable salt of glycopyrronium and/or beclometasone dipropionate (BDP).

The desiccant is contained in a bag able of being inserted in the desiccant chamber 12 or the desiccant is in the form of a single tablet able of being inserted in the desiccant chamber 12. The desiccant is or comprises molecular sieves made of a material with pores of uniform size, for instance alkaline salts of aluminosilicates, called zeolites, or aluminophosphates or porous glass or active carbon or artificial zeolites. The molecular sieves are configured to absorb small molecules such as molecules of water.

The dispensing device 9 comprises a metering device 14 having a dosing recess 15. The metering device 14 shown in the attached Figures comprises a shuttle 16 shaped like a plate and provided with said dosing recess 15.

The dispensing device 9 is movable, with respect to the container 7 and with respect to the inhalation channel 8, between an idle state (Figures 2A, 9 and 14), in which a dosing recess 15 is in communication with an opening 17 of the container 7 so as to be filled with a dose of the powdered medicament, and a triggered state (Figure 2C), in which the dosing recess 15 is in communication with the inhalation channel 8 for enabling inhalation of the dose of the powdered medicament contained in the dosing recess 15 through the mouthpiece 4.

The shuttle 16 is placed between the sub-assembly 10 and a bottom wall of the casing 2. The shuttle 16 is slidingly moveable between a filling position (Figures 2A, 9 and 14) and an inhalation position (Figures 2B and 2C). The filling position corresponds to the idle state of the metering device 14, in which the dosing recess 15 is in alignment with the opening 17 of the container 7 and faces said opening 17 so as to be filled with the dose of the powdered medicament. The inhalation position corresponds to an armed state (Figure 2B) which will be detailed later and to the triggered state (Figure 2C) of the metering device 14, in which the dosing recess 15 is in alignment with the inhalation channel 8.

The shuttle 16 is mechanically coupled to the cover 3 such that an opening of the cover 3 beyond a range of rotational movement from the closed position causes the shuttle 16 to move from the filling position to the inhalation position. Closing of the cover 3 causes the shuttle 16 to move back from the inhalation position to the filling position.

The metering device 14 further comprises a protective member 18 provided between the shuttle 16 and the inhalation channel 8. The protective member 18 is a plate arranged between the second opening of the inhalation channel 8 and the shuttle 16. The protective member 18 is parallel with respect to the shuttle 16 and is slidingly movable on or above the shuttle 16 between a closed position and an open position.

In the closed position, the protective member 18 is shifted backwards towards the second opening of the inhalation channel 8 and towards the container 7. In the closed position, a rear part of the protective member 18 may at least in part close the second opening of the inhalation channel 8. In the open position, the protective member 18 is shifted forward towards a wall of the casing 2. In the open position, a rear part of the protective member 18 leaves the second opening of the inhalation channel 8 open. The protective member 18 is in the closed position when the shuttle 16 is in the filling position (Figures 2A, 9 and 14). The protective member 18 may be moved between the closed position and the open position when the shuttle 16 is in the inhalation position (Figures 2B and 2C).

Therefore, the metering device 14 is configured to take the three different states cited above (idle, armed, triggered) and these states are determined by the positions of the shuttle 16 and of the protective member 18.

In the idle state (Figures 2A, 9 and 14), the shuttle 16 is in the filling position and the protective member 18 is in the closed position. The protective member 18 does not cover the dosing recess 15. The dosing recess 15 is communication with the opening of the container 7 to receive the medicament dose. In the armed state (Figure 2B), the shuttle 16 is in the inhalation position and the protective member 18 is in the closed position. The protective member 18 covers the dosing recess 15. The protective member 18 prevents the powdered medicament contained in the dosing recess 15 from entering the inhalation channel 8 and being lost in case of rotation or movement of the powder inhaler 1 in oblique position before the inhalation manoeuvre or if the user blows into the mouthpiece 4. In the triggered state (Figure 2C), the shuttle 16 is in the inhalation position and the protective member 18 is in the open position. The protective member 18 does not cover the dosing recess 15, thereby exposing the dosing recess 15 to the inhalation channel 8 so as to enable a user to inhale the dose of the powdered medicament contained in the dosing recess 15.

The dispensing device 9 further comprises a breath or inhalation actuated mechanism 19 coupled to the protective member 18. The inhalation actuated mechanism 19 comprises an inhalation actuated member 20 shaped like a flap, a coupling member 21 and a resilient element 22 (spring) arranged on the coupling member 21. The flap 20 is coupled to the protective member 18 through the coupling member 21 such that, if there is an inhalation suction force exceeding a predetermined value, the flap 20 is moved from a first position to a second position, thereby causing the protective member 18 to move from the closed position to the open position. The flap 20 is placed inside the casing 2 and close to the air inlets 5. In the first position (Figures 2A, 9 and 14), the flap 20 separates the air inlets 5 from the inhalation channel 8 and seats in a main airflow path. The flap 20 provides a resistance if the user blows into the device giving positive feedback. In the second position, (Figure 2C) the flap 20 is rotated with respect to the first position to open the air inlets 5 and to allow air flowing through the air inlets 5 into the inhalation channel 8 and out of the mouthpiece 4. The resilient element 22 is arranged such that said resilient element 22 holds the flap 20 in its first position. When the shuttle 16 is pushed forward by opening the cover 3, the resilient element 22 is compressed and charged and the reset force exerted on the flap 20 is released, so that the flap 20 can pivot or rotate from the first position into the second position that is pivoted downward relative to the first position if there is a sufficient high inhalation suction force in the inhalation channel 8.

The flap 20 is hinged to the casing 2 in order to rotate between the first position and the second position around a respective rotation axis which is substantially perpendicular to a main axis Z-Z of the inhalation channel 8. The coupling member 21 is also hinged to the casing 2 in order to rotate between a respective first position and second position around a respective rotation axis Y-Y which is substantially perpendicular to the main axis Z-Z of the inhalation channel 8.

The coupling member 21 comprises an arm, not shown, protruding towards the flap 20 and engaged with the flap 20 such that the clockwise rotation of the flap 20 from the first position to the second position causes a counterclockwise rotation of the coupling member 21 from its respective first position towards its respective second position.

The coupling member 21 comprises a prolongation 23 engaging with an opening formed in the protective member 18 in order to move the protective member 18 from the closed position to the open position when the coupling member 21 moves from its respective first position to its respective second position and vice-versa.

The prolongation 23 of the coupling member 21 is also moveably arranged in a longitudinal opening 24 which is formed in the shuttle 16 along its longitudinal direction, such that said prolongation 23 can freely move in the longitudinal opening 24, while a movement of the shuttle 16 from the inhalation position to the filling position causes the prolongation 23 of the coupling member 21 to abut against an edge of the longitudinal opening 24 thereby moving the coupling member 21 back into its initial first position.

The inhaler disclosed in the attached Figures further comprises a de-agglomerator arrangement 25 that is coupled to the second end of the inhalation channel 8 opposite the mouthpiece 4. The de-agglomerator arrangement 25 delimits a vortex chamber 26 and is constructed such that it generates a cyclonic airflow resulting in a strong velocity gradient. The protective member 18 is slidable on the shuttle 16 between its closed position, in which is covers the dosing recess 15, and its open position, in which it exposes the dosing recess 15 to the de-agglomerator arrangement 25 and the inhalation channel 8 when the metering member 14 is in the inhalation position, so that the dose of the powdered medicament can be inhaled through the de-agglomerator arrangement 25 and the inhalation channel 8 as well as the mouthpiece 4.

The internal mechanisms and functioning of the powder inhaler 1 disclosed here above are similar to those disclosed in documents WO 2004/012801, WO 2016/000983, WO2021/105440 by the same Applicant.

The powder inhaler 1 shown in the attached Figures may also comprise a sealing element 27 housed in the cover 3 and made of a material (e.g. silicone or a thermoplastic elastomer (TPE)) that is more deformable than a material of the mouthpiece 4, so that, when the cover 3 is engaged with the casing 2 and closes the mouthpiece 4 (Figures 2A, 9 and 14), a first portion of the sealing element 27 is coupled to the opening 6 of the mouthpiece 4 to tight close said opening 6 of the mouthpiece 4 and a second portion of the sealing element 27 is coupled to the air inlets 5 to tight close said air inlets 5.

The powder inhaler 1 may also comprise a dose counting unit, not shown in the embodiment of the attached drawings, contained into the casing 2 and coupled both to the inhalation actuated mechanism 19 and to the closure of the cover 3 after an efficacious inhalation has occurred. The casing 2 may also comprise a window or an opening for displaying the number of doses taken or the number of doses left in the container 7, this number being counted by the dose counting unit. For instance, the dose counting unit is the same or similar to the one disclosed in document WO 2004/012801.

According to the present invention, the powder inhaler 1 further comprises a sealing device 28 operationally active at a coupling zone of the shuttle 16 with the container 7 when the shuttle 16 is in the filling position, said coupling zone circumscribing the dosing recess 15 and the opening 17 of the container 7 and being interposed between the shuttle 16 and the container 7. The sealing device 28 removes or reduces gaps between the shuttle 16 and the container 7 and allows to prevent or at least reduce entry of moisture in the container 7 when the shuttle 16 is in the filling position.

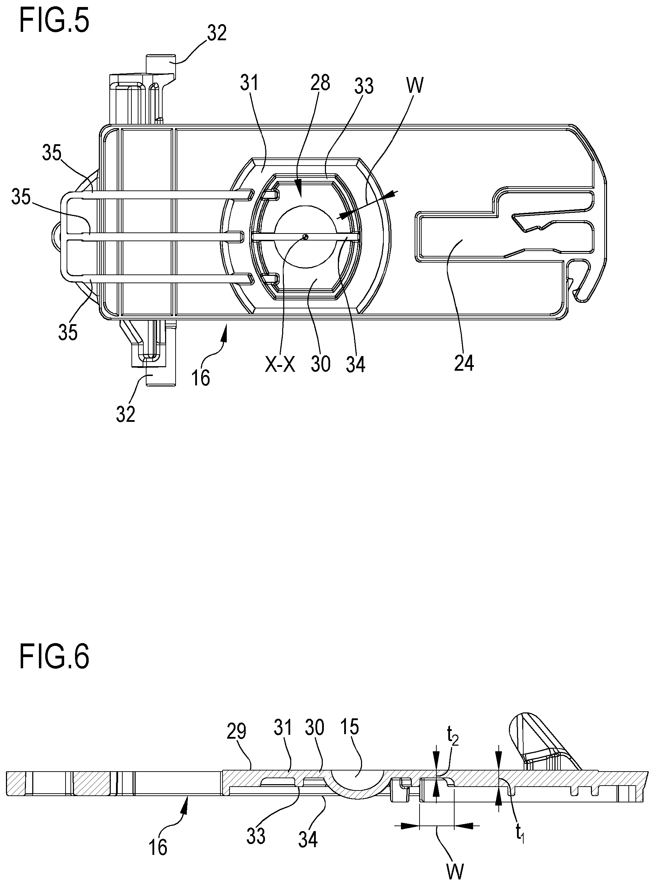

Figures 2A to 6 show a first illustrative embodiment of the sealing device 28 according to the invention.

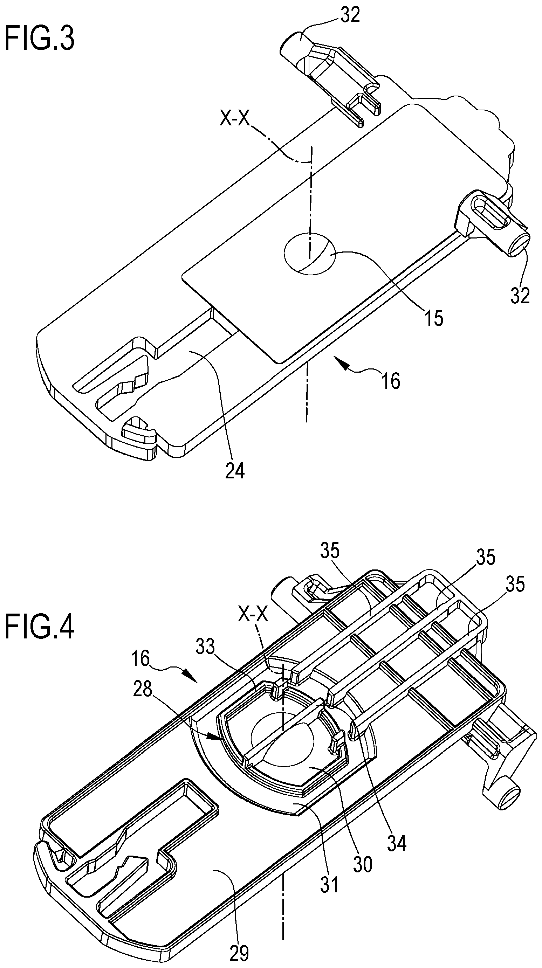

As can be seen in Figures 3, 4, 5 and 6, the shuttle 16 is shaped like a plate made of a single piece of plastic, e.g. acrylonitrile butadiene styrene copolymer (ABS). The shuttle 16 comprises a main part 29, a dosing part 30 and a deformable portion 31 surrounding the dosing recess 15 and connecting the dosing part 30 to the main part 29. The cited sealing device 28 is defined or comprises said deformable portion 31.

The dosing part 30 comprises the dosing recess 15 which is fashioned in an upper face of the dosing part 30 and is shaped like a cup having a circular edge, i.e. is a spherical cap recess.

The main part 29 surrounds the dosing part 30 and is coupled to the casing 2 and/or to the sub-assembly 10 in order to shift between the filling position and the inhalation position. To this aim, the shuttle 16 comprises pins 32 protruding laterally from the main part 29 and accommodated in tracks of the casing 2 and/or to the sub-assembly 10, so as to be able to slide. The longitudinal opening 24 is also fashioned in the main part 29.

The deformable portion 31 is a flat portion shaped like a band surrounding the dosing recess 15 and the dosing part 30. The deformable portion 31 and the dosing part 30 have a second wall thickness t2 which is smaller than a first wall thickness t1 of the main part 29. For instance, the first wall thickness t1 is 1.1 mm and the second wall thickness t2 is 0.45 mm. Therefore, a ratio t2/t1 of the second wall thickness t2 to the first wall thickness t1 is about 0.4.

The deformable portion 31 extends radially with respect to a central axis X-X of the dosing recess 15 perpendicular to the shuttle 16 and has an average width wₐᵥ measured along radial directions, wherein the average width wₐᵥ is an average of a radial width w measured on the 360°. For instance, the average width wₐᵥ is about 1.9 mm and a ratio t2/wₐᵥ of the second wall thickness t2 to the average width wₐᵥ is 0.24.

The dosing part 30 is delimited by a peripheral stiffening rib 33 which is fashioned on a side of the shuttle 16 opposite the dosing recess 15 and is surrounded by the deformable portion 31. A further straight stiffening rib 34 is placed on the side of the dosing part 30 opposite the dosing recess 15, is aligned to a direction of movement of the shuttle 16, passes through the central axis X-X of the dosing recess 15 and connects to the peripheral stiffening rib 33. This straight stiffening rib 34 does not extend over the deformable portion 31 and over the main part 29. The shuttle 16 comprises further stiffening ribs 35 on the main part 29 which are not connected to the dosing part 30.

Therefore, thanks to the different wall thickness with respect to the main part 29 and to the stiffening ribs 33, 34, 35 on the dosing part 30 and main part 29, the deformable portion 31 is less stiff than the main part 29 and the dosing part 30 along a direction perpendicular to a plane in which the shuttle 16 lies, i.e. the plane perpendicular to the central axis X-X of the dosing recess 15.

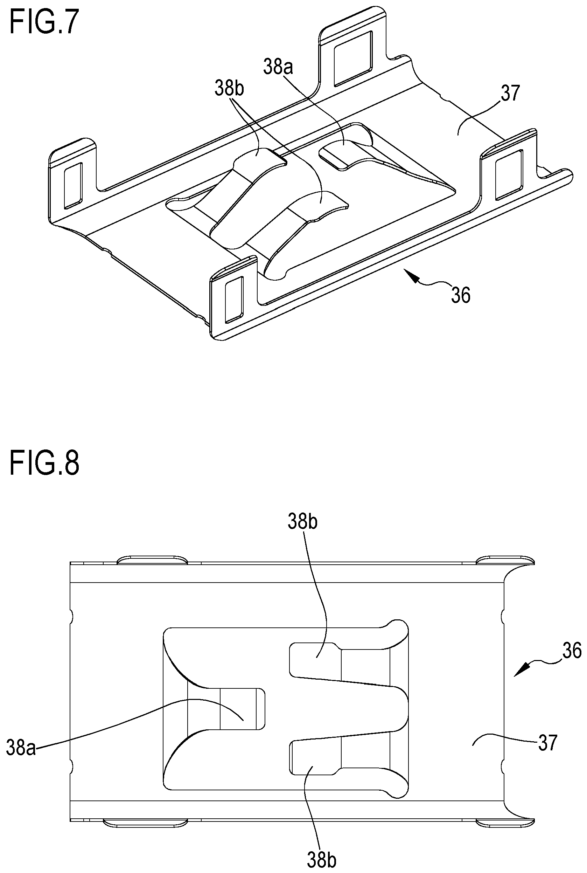

As shown in Figure 2A, a spring 36 is interposed between a bottom wall of the casing 2 and the dosing part 30 and is configured to push said dosing part 30 against the opening 17 of the container 7 by deforming the deformable portion 31 when the shuttle 16 is in the filling position. The spring 36 comprises a base 37 anchored to the casing 2 and/or to the sub-assembly 10 and three elastic arms 38a, 38b protruding from the base 37 towards the shuttle 16 (Figures 7 and 8). The shuttle 16 slides with respect to the spring 36 when moving between the filling position and the inhalation position.

When the shuttle 16 is in the filling position, a central arm 38a of said elastic arms 38a, 38b pushes against the straight stiffening rib 34 of the dosing part 30 at a center of the dosing recess 15 and two lateral arms 38b of said elastic arms 38a, 38b push against the peripheral stiffening rib 33, in order to press a zone of the dosing part 30 surrounding the dosing recess 15 against an edge 39 of the opening 17 of the container 7 (Figure 2A) by deforming the deformable portion 31.

Figures 9 to 13 show a variant of the first embodiment which differs from the first embodiment due to a different structure of the shuttle 16.

The deformable portion 31 of the shuttle 16 according to this variant comprises an elastomeric material, e.g. silicone or a thermoplastic elastomer (TPE) for medical - pharmaceutical applications, like styrene block copolymers (TPS (TPE-s)), thermoplastic polyolefin elastomers (TPO (TPE-o)), thermoplastic vulcanizates (TPV (TPE-v or TPV)), thermoplastic polyurethanes (TPU), thermoplastic copolyesters (TPC (TPE-E)), thermoplastic polyamides (TPA (TPE-A)), not classified thermoplastic elastomers (TPZ).

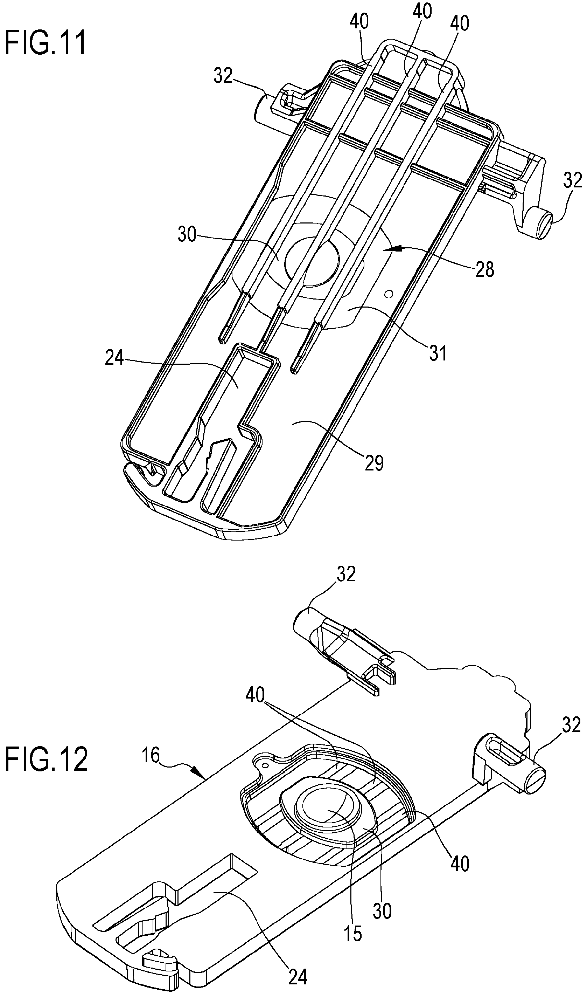

As better seen in Figures 11 and 12, the main part 29 and the dosing part 30 are spaced apart one from the other and connected to each other through connecting ribs 40 joining said main part 29 to the dosing part 30. The connecting ribs 40 are aligned to a direction of movement of the shuttle 16 between the filling position and the inhalation position and are positioned on a side of the shuttle 16 opposite the dosing recess 15. The main part 29, the dosing part 30 and the connecting ribs 40 are made of single piece of plastic, e.g. acrylonitrile butadiene styrene (ABS).

The deformable portion 31 of elastomeric material is co-molded or over-molded to the main part 29 and to the dosing part 30 and fills an annular gap between said main part 29 and dosing part 30. The main part 29 and the dosing part 30 have steps to accommodate the elastomeric material such that the deformable portion 31 is flush to an upper surface of the main part 29 and of the dosing part 30.

Also the deformable portion 31 of elastomeric material is a flat portion shaped like a band surrounding the dosing recess 15 and the dosing part 30. The deformable portion 31 and the dosing part 30 have a second wall thickness t2 which may be equal to (not shown in Figure 13) or smaller than the first wall thickness t1 of the main part 29.

For instance, the first wall thickness t1 and the second wall thickness t2 are the same, e.g. equal to 1.1 mm.

The deformable portion 31 extends radially with respect to a central axis X-X of the dosing recess 15 perpendicular to the shuttle 16 and has an average width wₐᵥ measured along radial directions, wherein the average width wₐᵥ is an average of a radial width w measured on the 360°. For instance, the average width wₐᵥ of the deformable portion 31 of elastomeric material is about 1.9 mm and a ratio t2/wₐᵥ of the second wall thickness t2 to the average width wₐᵥ is about 0.6.

Figures 14 to 17 show an embodiment of the inhaler 1 not part of the present invention in which the sealing device 28 is not integrated in the shuttle 16 but, differently, is a gasket 41 placed at the coupling zone which circumscribes the dosing recess 15 and the opening 17 of the container 7 and is interposed between the shuttle 16 and the container 7.

In this embodiment, the inhaler 1 comprises a support plate 42 made of a plastic material such as polycarbonate (PC), polypropylene (PP), acrylonitrile-butadienestyrene copolymer (ABS) or cyclic olefin copolymer (COC). The support plate 42 is sandwiched between the container 7 and the shuttle 16. The support plate 42 is anchored to the casing 2 and/or to the sub-assembly 10 so that it is stationary with respect to these elements. The support plate 42 has a through opening 43 and a through inhalation passage 44.

The through opening 43 faces the opening 17 of the container 7. The gasket 41 is made of an elastomeric material, e.g. a thermoplastic elastomer (TPE) or silicone, and is co-molded with or over-molded to an edge of the through opening 43.

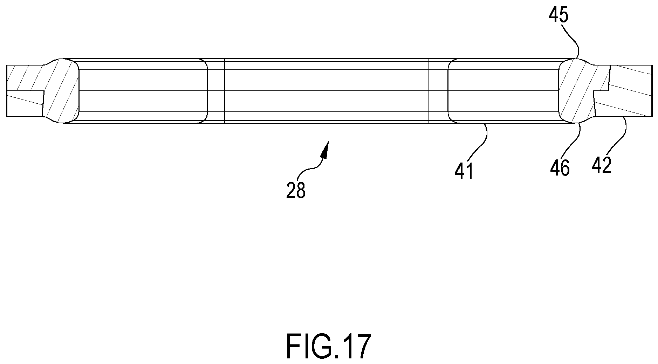

The cross section of Figure 17 shows that the gasket 41 has a raised bead 45 protruding towards the container 7 and a raised bead 46 protruding towards the shuttle 16.

The shuttle 16 may be the same or similar to the one disclosed in WO 2004/012801, WO 2016/000983 or WO2021/105440. The arms 38a, 38b of the spring 36 pushes the shuttle 16 against the support plate 42 and pushes the support plate 42 against the sub-assembly 10, so that the gasket 41 is sandwiched between the shuttle 16 and the casing 2 and seals the opening 17.

### Example

The different embodiments were tested.

Test conditions: 30°C and 75% RH.

The desiccant was removed from the desiccant chamber 12 and the medicament chamber 11 was filled with 3g of calcium chloride anhydrous to evaluate the moisture ingress in said medicament chamber 11.

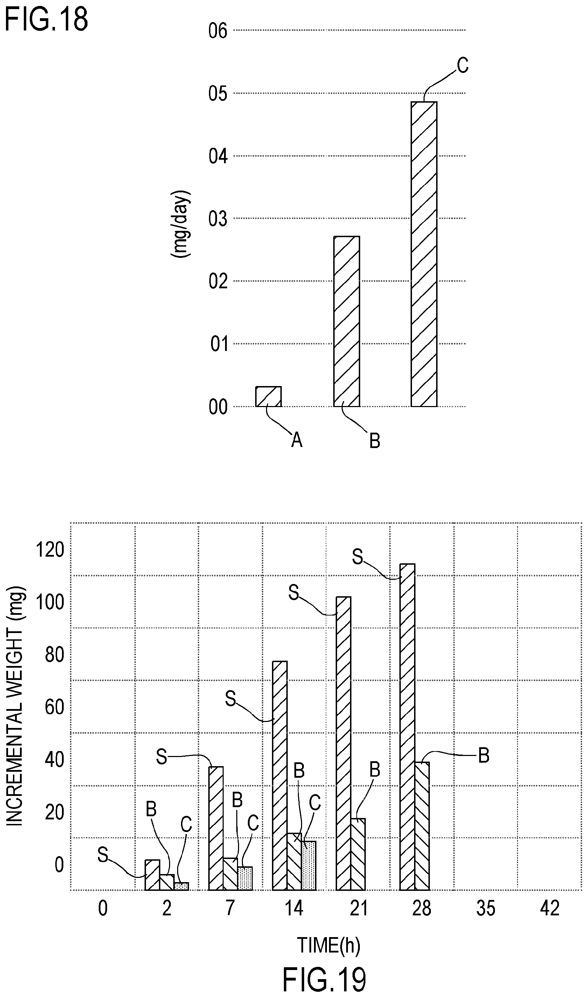

Fig.18 shows a differential weight per day (mg/day) due to moisture absorption of the different embodiments/variants with respect to a standard powder inhaler, wherein:
A - first embodiment of the invention of Figures 2A to 6;
B - variant of the invention of Figures 9 to 13;
C - embodiment of Figures 14 to 17 not part of the present invention.

Fig.19 is a chart showing a cumulative/incremental weight (mg) over time (h) due to moisture absorption in a standard powder inhaler and in the embodiments/variants, wherein:
S - powder inhaler with standard shuttle as disclosed in WO2004/012801 or WO 2016/000983;
B - variant of the invention of Figures 9 to 13;
C - embodiment of Figures 14 to 17 not part of the present invention.

As shown in Figure 18, all the embodiments/variants A, B and C exhibited an improvement in the humidity protection with respect to the standard powder inhaler.

As shown in Figure 19, after 14 days, both the embodiments/variants B and C showed a saving of about 70 mg of water and after 28 days the variant B show a saving of about 75 mg of water.

### List of parts

powder inhaler 1
casing 2
cover 3
mouthpiece 4
air inlets 5
opening 6 of the mouthpiece
container 7
inhalation channel 8
dispensing device 9
sub-assembly 10
medicament chamber 11
desiccant chamber 12
permeable membrane 13
metering device 14
dosing recess 15
shuttle 16
opening 17 of the container
protective member 18
breath or inhalation actuated mechanism 19
inhalation actuated member or flap 20
coupling member 21
resilient element 22
prolongation 23
longitudinal opening 24
de-agglomerator arrangement 25
vortex chamber 26
a sealing element 27
sealing device 28
main part 29
dosing part 30
deformable portion 31
pins 32
peripheral stiffening rib 33
stiffening rib 34
further stiffening ribs 35
spring 36
base 37
elastic arms 38a, 38b
edge 39 of the opening of the container
connecting ribs 40
gasket 41
support plate 42
through opening 43
through inhalation passage 44
raised bead 45
raised bead 46
central axis X-X

## Claims

1. Powder inhaler, comprising:
- a casing (2) having a mouthpiece (4);
- an inhalation channel (8) housed in the casing (2) and connected to the mouthpiece (4);
- a container (7) housed in the casing (2) for storing a powdered medicament;
- a metering device (14) comprising a shuttle (16) having a dosing recess (15), wherein the shuttle (16) is movable between a filling position, in which the dosing recess (15) is in alignment with an opening (17) of the container (7) and faces said opening (17) so as to be filled with a dose of the powdered medicament, and an inhalation position, in which the dosing recess (15) is in alignment with the inhalation channel (8), for enabling inhalation of the dose of the powdered medicament contained in the dosing recess (15) through the mouthpiece (4);
wherein the powder inhaler (1) further comprises a sealing device (28) operationally active at a coupling zone of the shuttle (16) with the container (7) when the shuttle (16) is in the filling position, said coupling zone circumscribing the dosing recess (15) and the opening (17);
wherein the shuttle (16) comprises a main part (29) and a dosing part (30); wherein the dosing part (30) comprises the dosing recess (15);
**characterized in that** the sealing device (28) comprises a deformable portion (31) surrounding the dosing recess (15) and connecting the dosing part (30) to the main part (29); wherein the deformable portion (31) is less stiff than the main part (29) along a direction perpendicular to a lying plane of the shuttle (16); wherein at least one spring (36) is interposed between the casing (2) and the dosing part (30) and is configured to push said dosing part (30) against the opening (17) of the container (7) by deforming the deformable portion (31) when the shuttle (16) is in the filling position.

2. The inhaler of claim 1, wherein the deformable portion (31) is shaped like a band surrounding the dosing recess (15).

3. The inhaler of claim 1 or 2, wherein the shuttle (16) is shaped like a plate and the main part (29) has a first wall thickness (t1), wherein the deformable portion (31) has a second wall thickness (t2) equal to or smaller than the first wall thickness (t1).

4. The inhaler of claim 3, wherein a ratio t2/t1 of the second wall thickness (t2) to the first wall thickness (t1) is smaller than 0.9, optionally smaller than 0.5; wherein said ratio t2/t1 of the second wall thickness (t2) to the first wall thickness (t1) is greater than 0.1, optionally greater than 0.4.

5. The inhaler of any of claims 1 to 4, wherein the dosing part (30) comprises a peripheral stiffening rib (33) surrounded by the deformable portion (31).

6. The inhaler of claim 5, the peripheral stiffening rib (33) is fashioned on a side of the shuttle (16) opposite the dosing recess (15).

7. The inhaler of any of claims 1 to 6, wherein the deformable portion (31) extends radially with respect to a central axis (X-X) of the dosing recess (15) perpendicular to the shuttle (16) and has an average width (wₐᵥ) measured along radial directions.

8. The inhaler of claim 7, when depending on claim 3, wherein a ratio t2/wₐᵥ of the second wall thickness (t2) to the average width (wₐᵥ) is smaller than 0.4.

9. The inhaler of any of claims 1 to 8, wherein the main part (29), the deformable portion (31) and the dosing part (30) are made in a single piece.

10. The inhaler of claim 9, wherein the single piece is made of plastic, optionally of at least one rigid thermoplastic material.

11. The inhaler of any of claims 1 to 8, wherein the deformable portion (31) is made of or comprises an elastomeric material.

12. The inhaler of any of claims 1 to 11, wherein the dosing part (30) comprises at least one stiffening rib (34) on a side of the shuttle (16) opposite the dosing recess (15).

13. The inhaler of claim 12, wherein the at least one stiffening rib (34) is straight and passes through a central axis (X-X) of the dosing recess (15).

14. The inhaler of any of claims 1 to 13, wherein the shuttle (16) comprises at least one connecting rib (40) joining the main part (29) to the dosing part (30).

15. The inhaler of any of claims 1 to 14, wherein the at least one spring (36) contacts the dosing part (30) at least at a center of the dosing recess (15).

## Patentansprüche

1. Pulverinhalator, umfassend:
- ein Gehäuse (2) mit einem Mundstück (4);
- einen Inhalationskanal (8), welcher in dem Gehäuse (2) untergebracht und mit dem Mundstück (4) verbunden ist;
- einen Behälter (7), welcher in dem Gehäuse (2) untergebracht ist, zum Lagern eines pulverförmigen Medikaments;
- eine Dosiervorrichtung (14) umfassend ein Shuttle (16) mit einer Dosieraussparung (15), wobei das Shuttle (16) beweglich ist zwischen einer Füllposition, in welcher die Dosieraussparung (15) in Flucht mit einer Öffnung (17) des Behälters (7) steht und der Öffnung (17) zugewandt ist, um mit einer Dosis des pulverförmigen Medikaments befüllt zu werden, und einer Inhalationsposition, in welcher die Dosieraussparung (15) in Flucht mit dem Inhalationskanal (8) steht, um Inhalation der Dosis des pulverförmigen Medikaments, welche in der Dosieraussparung (15) enthalten ist, durch das Mundstück (4) zu ermöglichen;
wobei der Pulverinhalator (1) ferner eine Versiegelungsvorrichtung (28) umfasst, welche an einer Kopplungszone des Shuttles (16) mit dem Behälter (7) betriebswirksam ist, wenn sich das Shuttle (16) in der Füllposition befindet, wobei die Kopplungszone die Dosieraussparung (15) und die Öffnung (17) umschreibt;
wobei das Shuttle (16) einen Hauptteil (29) und einen Dosierteil (30) umfasst; wobei der Dosierteil (30) die Dosieraussparung (15) umfasst;
**dadurch gekennzeichnet, dass** die Versiegelungsvorrichtung (28) einen verformbaren Abschnitt (31) umfasst, welcher die Dosieraussparung (15) umgibt und den Dosierteil (30) mit dem Hauptteil (29) verbindet; wobei der verformbare Abschnitt (31) entlang einer Richtung senkrecht zu einer Liegeebene des Shuttles (16) weniger steif ist als der Hauptteil (29); wobei wenigstens eine Feder (36) zwischen dem Gehäuse (2) und dem Dosierteil (30) angeordnet ist und dazu eingerichtet ist, den Dosierteil (30) gegen die Öffnung (17) des Behälters (7) durch Verformen des verformbaren Abschnitts (31) zu drücken, wenn sich das Shuttle (16) in der Füllposition befindet.

2. Inhalator nach Anspruch 1, wobei der verformbare Abschnitt (31) wie ein Band geformt ist, welches die Dosieraussparung (15) umgibt.

3. Inhalator nach Anspruch 1 oder 2, wobei das Shuttle (16) wie eine Platte geformt ist und der Hauptteil (29) eine erste Wanddicke (t1) aufweist, wobei der verformbare Abschnitt (31) eine zweite Wanddicke (t2) aufweist, welche gleich oder kleiner als die erste Wanddicke (t1) ist.

4. Inhalator nach Anspruch 3, wobei ein Verhältnis t2/t1 der zweiten Wanddicke (t2) zu der ersten Wanddicke (t1) kleiner als 0,9, gegebenenfalls kleiner als 0,5 ist; wobei das Verhältnis t2/t1 der zweiten Wanddicke (t2) zu der ersten Wanddicke (t1) größer als 0,1, gegebenenfalls größer als 0,4 ist.

5. Inhalator nach einem der Ansprüche 1 bis 4, wobei der Dosierteil (30) eine periphere Versteifungsrippe (33) umfasst, welche von dem verformbaren Abschnitt (31) umgeben ist.

6. Inhalator nach Anspruch 5, wobei die periphere Versteifungsrippe (33) auf einer Seite des Shuttles (16) gegenüber der Dosieraussparung (15) ausgebildet ist.

7. Inhalator nach einem der Ansprüche 1 bis 6, wobei sich der verformbare Abschnitt (31) radial in Bezug auf eine Mittelachse (X-X) der Dosieraussparung (15) senkrecht zu dem Shuttle (16) erstreckt und eine durchschnittliche Breite (wₐᵥ) aufweist, welche entlang radialer Richtungen gemessen ist.

8. Inhalator nach Anspruch 7, wenn er von Anspruch 3 abhängt, wobei ein Verhältnis t2/wₐᵥ der zweiten Wanddicke (t2) zu der durchschnittlichen Breite (wₐᵥ) kleiner als 0,4 ist.

9. Inhalator nach einem der Ansprüche 1 bis 8, wobei der Hauptteil (29), der verformbare Abschnitt (31) und der Dosierteil (30) als ein einziges Stück hergestellt sind.

10. Inhalator nach Anspruch 9, wobei das einzige Stück aus Kunststoff hergestellt ist, gegebenenfalls aus wenigstens einem starren thermoplastischen Material.

11. Inhalator nach einem der Ansprüche 1 bis 8, wobei der verformbare Abschnitt (31) aus einem Elastomermaterial hergestellt ist oder dieses umfasst.

12. Inhalator nach einem der Ansprüche 1 bis 11, wobei der Dosierteil (30) wenigstens eine Versteifungsrippe (34) auf einer Seite des Shuttles (16) gegenüber der Dosieraussparung (15) umfasst.

13. Inhalator nach Anspruch 12, wobei die wenigstens eine Versteifungsrippe (34) gerade ist und durch eine Mittelachse (X-X) der Dosieraussparung (15) verläuft.

14. Inhalator nach einem der Ansprüche 1 bis 13, wobei das Shuttle (16) wenigstens eine Verbindungsrippe (40) umfasst, welche den Hauptteil (29) an den Dosierteil (30) anfügt.

15. Inhalator nach einem der Ansprüche 1 bis 14, wobei die wenigstens eine Feder (36) den Dosierteil (30) wenigstens in einer Mitte der Dosieraussparung (15) berührt.

## Revendications

1. Inhalateur de poudre, comprenant :
- un boîtier (2) ayant un embout buccal (4) ;
- un canal d'inhalation (8) logé dans le boîtier (2) et raccordé à l'embout buccal (4) ;
- un récipient (7) logé dans le boîtier (2) pour stocker un médicament en poudre ;
- un dispositif de mesure (14) comprenant une navette (16) ayant un renfoncement de dosage (15), dans lequel la navette (16) est mobile entre une position de remplissage, dans laquelle le renfoncement de dosage (15) se trouve en alignement avec une ouverture (17) du récipient (7) et qui fait face à ladite ouverture (17) afin d'être rempli avec une dose du médicament en poudre, et une position d'inhalation, dans laquelle le renfoncement de dosage (15) se trouve en alignement avec le canal d'inhalation (8), pour permettre une inhalation de la dose du médicament en poudre contenue dans le renfoncement de dosage (15) à travers l'embout buccal (4) ;
dans lequel l'inhalateur de poudre (1) comprend en outre un dispositif d'étanchéité (28) fonctionnellement actif au niveau d'une zone d'accouplement de la navette (16) avec le récipient (7) lorsque la navette (16) se trouve dans la position de remplissage, ladite zone d'accouplement entourant le renfoncement de dosage (15) et l'ouverture (17) ;
dans lequel la navette (16) comprend une partie principale (29) et une partie de dosage (30) ; dans lequel la partie de dosage (30) comprend le renfoncement de dosage (15) ;
**caractérisé en ce que** le dispositif d'étanchéité (28) comprend une portion déformable (31) entourant le renfoncement de dosage (15) et raccordant la partie de dosage (30) à la partie principale (29) ; dans lequel la portion déformable (31) est moins rigide que la partie principale (29) le long d'une direction perpendiculaire à un plan de repos de la navette (16) ; dans lequel au moins un ressort (36) est interposé entre le boîtier (2) et la partie de dosage (30) et est configuré pour pousser ladite partie de dosage (30) contre l'ouverture (17) du récipient (7) en déformant la portion déformable (31) lorsque la navette (16) se trouve dans la position de remplissage.

2. Inhalateur selon la revendication 1, dans lequel la portion déformable (31) est mise sous forme de bande entourant le renfoncement de dosage (15).

3. Inhalateur selon la revendication 1 ou 2, dans lequel la navette (16) est mise sous forme de plaque et la partie principale (29) présente une première épaisseur de paroi (t1), dans lequel la portion déformable (31) présente une seconde épaisseur de paroi (t2) inférieure ou égale à la première épaisseur de paroi (t1).

4. Inhalateur selon la revendication 3, dans lequel un ratio t2/t1 de la seconde épaisseur de paroi (t2) à la première épaisseur de paroi (t1) est inférieur à 0,9, éventuellement inférieur à 0,5 ; dans lequel ledit ratio t2/t1 de la seconde épaisseur de paroi (t2) à la première épaisseur de paroi (t1) est supérieur à 0,1, éventuellement supérieur à 0,4.

5. Inhalateur selon l'une quelconque des revendications 1 à 4, dans lequel la partie de dosage (30) comprend une nervure de raidissement périphérique (33) entourée de la portion déformable (31).

6. Inhalateur selon la revendication 5, la nervure de raidissement périphérique (33) est façonnée sur un côté de la navette (16) opposé au renfoncement de dosage (15).

7. Inhalateur selon l'une quelconque des revendications 1 à 6, dans lequel la portion déformable (31) s'étend radialement par rapport à un axe central (X-X) du renfoncement de dosage (15) perpendiculaire à la navette (16) et présente une largeur moyenne (wₐᵥ) mesurée le long des directions radiales.

8. Inhalateur selon la revendication 7, lorsqu'elle dépend de la revendication 3, dans lequel un ratio t2/wₐᵥ de la seconde épaisseur de paroi (t2) à la largeur moyenne (wₐᵥ) est inférieur à 0,4.

9. Inhalateur selon l'une quelconque des revendications 1 à 8, dans lequel la partie principale (29), la portion déformable (31) et la partie de dosage (30) sont constituées d'une pièce unique.

10. Inhalateur selon la revendication 9, dans lequel la pièce unique est constituée de matière plastique, éventuellement d'au moins un matériau thermoplastique rigide.

11. Inhalateur selon l'une quelconque des revendications 1 à 8, dans lequel la portion déformable (31) est constituée ou comprend un matériau élastomère.

12. Inhalateur selon l'une quelconque des revendications 1 à 11, dans lequel la partie de dosage (30) comprend au moins une nervure de raidissement (34) sur un côté de la navette (16) opposé au renfoncement de dosage (15).

13. Inhalateur selon la revendication 12, dans lequel la au moins une nervure de raidissement (34) est droite et passe à travers un axe central (X-X) du renfoncement de dosage (15).

14. Inhalateur selon l'une quelconque des revendications 1 à 13, dans lequel la navette (16) comprend au moins une nervure de raccordement (40) joignant la partie principale (29) à la partie de dosage (30).

15. Inhalateur selon l'une quelconque des revendications 1 à 14, dans lequel le au moins un ressort (36) entre en contact avec la partie de dosage (30) au moins au niveau d'un centre du renfoncement de dosage (15).
